# FASCICULE DE BREVET EUROPEEN

(11) **EP 1 463 816 B1**
(45) Date de publication et mention de la délivrance du brevet: **29.08.2007**
(21) Numéro de dépôt: 02804929.4
(22) Date de dépôt: 17.12.2002
(51) Int. Cl.: C12N 15/10, C12N 15/01, C07K 14/62, C12N 15/90

(54) **PROCEDE POUR L INDUCTION MAITRISEE DE MUTATIONS SOMATIQUES E T SON APLLICATION EN PROTEOMIQUE**
VERFAHREN ZUR KONTROLLIERTEN INDUKTION SOMATISCHER MUTATIONEN UND VERWENDUNG DAVON IN PROTEOMIK
METHOD FOR CONTROLLED INDUCTION OF SOMATIC MUTATIONS AND USE THEREOF IN PROTEOMICS

(30) Priorité: 18.12.2001 FR 0116352; 08.08.2002 FR 0210078
(43) Date de publication de la demande: 06.10.2004
(73) Titulaire: Mixis France S.A., 75014 Paris (FR)
(72) Inventeur: REYNAUD, Claude-Agnès, F-75015 Paris (FR); WEILL, Jean-Claude, F-75015 Paris (FR)
(74) Mandataire: Crawley, Karen Anne
(86) Numéro de dépôt international: PCT/FR2002/004375
(87) Numéro de publication internationale: WO 2003/052098

(56) Documents cités:
- WO-A-00/22111
- US-A- 5 885 827
- FAILI AHMAD ET AL: "Induction of somatic hypermutation in immunoglobulin genes is dependent on DNA polymerase iota." NATURE (LONDON), vol. 419, no. 6910, 2002, pages 944-947, XP002245732 31 October, 2002 ISSN: 0028-0836
- FAILI AHMAD ET AL: "AID-dependent somatic hypermutation occurs as a DNA single-strand event in the BL2 cell line." NATURE IMMUNOLOGY, vol. 3, no. 9, septembre 2002 (2002-09), pages 815-821, XP008014204 ISSN: 1529-2908
- LUXEMBOURG ALAIN T ET AL: "Modulation of signaling via the B cell antigen receptor by C21, the receptor for C3dg and EBV." JOURNAL OF IMMUNOLOGY, vol. 153, no. 10, 1994, pages 4448-4457, XP002211126 ISSN: 0022-1767
- YELAMOS J ET AL: "Targeting of non-lg sequences in place of the V segment by somatic hypermutation." NATURE (LONDON), vol. 376, no. 6537, 1995, pages 225-229, XP002211125 ISSN: 0028-0836 cité dans la demande
- POLTORATSKY V ET AL: "Expression of error-prone polymerases in BL2 cells activated for Ig somatic hypermutation." PROCEEDINGS OF THE NATIONAL ACADEMY OF SCIENCES OF THE UNITED STATES OF AMERICA. UNITED STATES 3 JUL 2001, vol. 98, no. 14, 3 juillet 2001 (2001-07-03), pages 7976-7981, XP002245865 ISSN: 0027-8424 cité dans la demande
- DENEPOUX S ET AL: "INDUCTION OF SOMATIC MUTATION IN A HUMAN B CELL LINE IN VITRO" IMMUNITY, CELL PRESS, US, vol. 6, no. 6, janvier 1997 (1997-01), pages 35-46, XP000876832 ISSN: 1074-7613 cité dans la demande
- WELLER S ET AL: "CD40-CD40L independent Ig gene hypermutation suggests a second B cell diversification pathway in humans" PROCEEDINGS OF THE NATIONAL ACADEMY OF SCIENCES OF USA, NATIONAL ACADEMY OF SCIENCE. WASHINGTON, US, vol. 98, no. 3, 30 janvier 2001 (2001-01-30), pages 1166-1170, XP002182729 ISSN: 0027-8424
- GERGELY LAJOS ET AL: "A simplified method for Ca-2+ flux measurement on isolated human B cells that uses flow cytometry." CLINICAL AND DIAGNOSTIC LABORATORY IMMUNOLOGY, vol. 4, no. 1, 1997, pages 70-74, XP001104516 ISSN: 1071-412X
- REYNAUD C-A ET AL: "Mismatch repair and immunoglobulin gene hypermutation: did we learn something?" IMMUNOLOGY TODAY, ELSEVIER PUBLICATIONS, CAMBRIDGE, GB, vol. 20, no. 11, novembre 1999 (1999-11), pages 522-527, XP004183761 ISSN: 0167-5699
- WEILL J-C ET AL: "Rearrangement/hypermutation/gene conversion: when, where and why?" IMMUNOLOGY TODAY, ELSEVIER PUBLICATIONS, CAMBRIDGE, GB, vol. 17, no. 2, 1 février 1996 (1996-02-01), pages 92-97, XP004034660 ISSN: 0167-5699

## Description

### DOMAINE DE L'INVENTION

La présente invention concerne la biologie, et plus spécialement le domaine de l'adaptation par mutation dirigée, spécifique au monde vivant. Elle concerne plus particulièrement l'induction de mutations somatiques in vitro, ainsi que les applications que les perfectionnements d'une telle technique rendent désormais possibles.

Dans une forme de réalisation particulière, l'invention concerne l'induction des mutations sur le lymphome de Burkitt BL2.

### ARRIÈRE-PLAN TECHNOLOGIQUE DE L'INVENTION

Pour simplifier, il est fait référence dans la suite au lymphome de Burkitt BL2. Il faut cependant souligner que cela vise seulement à faciliter l'exposé de la technique concernée et du concept inventif revendiqué, sans pour autant limiter l'invention à cet égard.

La lignée cellulaire du lymphome de Burkitt de type I BL2, présente les caractéristiques des cellules B centroblastiques. Cette lignée cellulaire est la plus proche contrepartie immortalisée des centroblastes des centres germinatifs. Son origine de centre germinatif a été confirmée par la présence de mutations somatiques dans les gènes V_{H} d'immunoglobuline des lymphomes de Burkitt.

L'homogénéité et la stabilité des cellules BL2 dans des cultures appropriées a été soulignée (Denépoux S. et al., Immunity, vol. 6, 35-46, 1997). Il a été proposé, en conformité avec le phénotype de centroblaste, de considérer que la BL2 résulte de la transformation d'un centroblaste de centre germinatif qui a subi plusieurs phases de mutations somatiques.

On estime par ailleurs que les cellules B subissent une hypermutation au stade du centroblaste (Pascual V. et al., J. Exp. Med. 180, 329-339 (1994).

Le phénomène d'hypermutation des immunoglobulines a lieu dans les centres germinatifs, après stimulation d'une cellule B par un antigène T-dépendant.

En particulier, Denépoux S. et al., (op. cité) ont établi que les cellules de lymphome, notamment les cellules BL2, peuvent enclencher un processus d'hypermutation après agrégation de leur récepteur de surface et co-culture avec une cellule T-auxiliaire ou amplificatrice, après une semaine de culture. Il a été conclu dans cet article que c'est par des activations répétées des cellules BL2 que l'on peut augmenter la fréquence de mutation. Egalement ces auteurs ont souligné que les mutations induites in vitro sur la BL2 n'affectent pas la région constante de l'IgM et ne font pas apparaître une sélection dirigée par un antigène.

Egalement, Yélamos J. et al., Nature, vol. 376, pp. 225-229 (1995) ont relaté des expériences tendant à montrer que le fragment de gène V de l'immunoglobuline n'est pas indispensable pour une hypermutation et que la construction de substrats de mutation artificiels pourrait s'en trouver simplifiée.

Luxembourg A.T. et al., Journal of Immunology, Vol. 153, No. 10, 1994, pp. 4448 - 4457, décrivent l'activation de cellules naïves par CD21 ou CD19, et l'augmentation du niveau de *c-fos*, qui est un proto-oncogène, par la co-activation par l'IgM de surface et le CD21.

Malgré ces développements, on ne dispose pas jusqu'ici de moyens efficaces pour étudier ces phénomènes et pour en retirer des résultats pratiques, en particulier à des fins de diagnostic et/ou de suivi, ainsi que de traitement des processus tumoraux, dans des conditions de praticité et de rapidité satisfaisantes.

Il y avait donc un besoin pour des moyens permettant de réaliser des tests d'hypermutation somatique rapides et fiables. Il existait plus particulièrement un besoin pour des moyens permettant de disposer d'un modèle d'induction de mutations somatiques, applicable aux gènes V, mais également aux autres gènes susceptibles d'être impliqués dans un processus tumoral, notamment chez les humains, comme par exemple les gènes Bcl-6 et Fas Ligand (FasL).

Or, on a maintenant trouvé que les cellules de lymphome, notamment les cellules BL2, peuvent enclencher un processus d'hypermutation accéléré, contrairement aux données de la technique antérieure, selon lesquelles un processus comparable était long et fastidieux à mettre en oeuvre.

Plus précisément, on a maintenant trouvé de manière inattendue que, avec pour modèle la cellule BL2, on peut réaliser in vitro dans des conditions de rapidité et d'efficacité exceptionnelles, l'induction de mutations somatiques dans des cellules appropriées en ajoutant aux cellules à faire muter, dans des conditions de culture et dans un milieu appropriés aux dites cellules, au moins une combinaison ternaire des anticorps anti-CD19, anti-CD21 et anti-IgM.

L'invention a ainsi pour premier objet un procédé pour l'induction de mutations somatiques in vitro ou ex vivo, comportant (1) l'adjonction aux cellules à faire muter, dans des conditions de culture et dans un milieu appropriés aux dites cellules, d'au moins une combinaison ternaire des anticorps anti-CD19, anti-CD21 et anti-IgM, tandis que (2) lesdits anticorps anti-IgM sont biotinylés et sont soumis à une agrégation spécifique par la streptavidine couplée à un support, avantageusement à un support composé en totalité ou en partie par des billes magnétiques.

Selon une caractéristique, l'induction de mutations somatiques selon l'invention est mise en oeuvre sur une courte durée, en pratique sur une durée de l'ordre de 1h30 (une heure et demie).

Selon une caractéristique avantageuse, le procédé selon l'invention ne comporte pas la mise en oeuvre de co-cultures avec des cellules d'un autre type cellulaire, notamment avec une cellule T.

On a en effet constaté que le procédé préconisé selon l'invention, dans lequel est effectuée une agrégation spécifique d'anticorps anti-IgM biotinylés par la streptavidine couplée à un support tel que, de préférence, des billes magnétiques, ainsi qu'une combinaison d'au moins les trois anticorps anti-CD19, anti-CD21 et anti-IgM, procure in vitro une mutation rapide de cellules cultivées en présence de ces anticorps, dans un temps très court qui n'a aucune mesure avec les durées indiquées pour l'obtention de mutations in vitro selon la technique antérieure.

Selon une variante de réalisation, le procédé selon l'invention peut comporter une combinaison d'anticorps anti-IgM choisie parmi une combinaison des anticorps anti-IgM et anti-CD19, une combinaison des anticorps anti-IgM et anti-CD21, et leurs mélanges en toutes proportions.

Selon une autre caractéristique, le procédé selon l'invention est mis en oeuvre pour l'induction de mutations sur le lymphome de Burkitt BL2.

L'invention a également pour objet l'utilisation de ce procédé d'induction de mutations somatiques pour la réalisation de tests in vitro d'hypermutagénicité au niveau protéique et/ou génique.

Selon une forme dé réalisation, cette utilisation est appliquée aux lymphomes B, en particulier aux lymphomes B humains, plus particulièrement pour l'induction de mutations sur le lymphome de Burkitt BL2.

Selon une forme de réalisation particulièrement préférée, le système selon l'invention est utilisé pour l'induction de mutations sur le lymphome de Burkitt BL2 dans la phase G0/G1.

L'invention a également pour objet un kit pour l'induction de mutations somatiques, caractérisé en ce qu'il comporte un système inducteur de mutations somatiques tel que défini plus haut.

Un autre objet de l'invention est l'utilisation de ce kit pour l'identification qualitative et/ou quantitative de composants du mutasome, notamment par l'analyse des protéines.

Dans une forme de réalisation, l'utilisation du kit selon l'invention comporte l'identification des modifications post-traductionnelles induites, en particulier par identification, isolement et analyse d'un composant du mutasome, notamment d'un composant protéinique, qui apparaît pendant ladite induction.

Selon une caractéristique de mise en oeuvre préférée, cette utilisation comporte la fourniture d'au moins un gène, notamment un gène codant pour une protéine d'intérêt, sur lequel on souhaite induire des mutations, tandis que ledit gène est inclus dans une cassette contenant le promoteur et l'activateur (enhancer) des gènes codant pour la chaîne lourde ou légère des IgG, et que ledit gène est transfecté dans des cellules de lymphome, notamment de lymphome de Burkitt BL2.

Selon une forme de réalisation avantageuse, pour faire muter une séquence quelconque, on encadre la séquence à faire muter par le promoteur et l'activateur des Ig, de préférence dans une cassette de mutation.

L'invention est décrite ci-après en référence à un exemple de réalisation, qui est purement illustratif et qui vise à procurer à l'homme du métier des indications pratiques pour la réalisation de l'invention. Sur la base de ces indications et de ses connaissances propres, l'homme du métier est ainsi apte à concevoir, sans sortir du cadre de la présente invention, des systèmes de test conformes à l'invention revendiquée.

Pour l'induction de mutations somatiques in vitro sur la cellule BL2, on a procédé comme suit:

### - Conditions de culture de la cellule BL2

On a fourni des cellules BL2 et on les a maintenues dans un milieu complet à raison de 10⁵ cellules par ml, pendant 24 h à 37°C et à 5% de CO₂.

Le milieu de culture était du milieu RPMI 1640, complété avec 10% de FCS, ainsi que 100 U/ml (unités/ml) de pénicilline et 100 µg/ml de streptomycine.

### - Stimulation

On a lavé les cellules dans du milieu RPMI, pour enlever toute trace du sérum, par centrifugation à environ 1500 tours/min pendant 7 minutes, à une température comprise entre 4 et 10°C.

On a repris les cellules BL2 dans du milieu RPMI à raison de 10⁶ cellules pour 500 µl.

Dans un flacon de 15 ml, on a introduit 10⁶ cellules dans de la glace, et on a ajouté:
· 20 µl d'anti-CD19 couplé à du FITC (fourni par Immunotech),
· 20 µl d'anti-CD21 couplé à la PE (fourni par Becton Dickinson) et
· 4 µl d'anti-IgM couplé à la biotine (fourni par Immunotech).

Après agitation jusqu'à obtention d'un mélange homogène, on a placé celui-ci sur de la glace et on l'y a laissé pendant environ 20 minutes.

On a ensuite lavé les cellules de ce mélange dans 10 ml de milieu RPMI (à 4°C), puis on a soumis à une centrifugation à 1500 tours/min pendant 7 min. On a repris les cellules ainsi lavées dans 500 µl de RPMI dans un tube d'Eppendorf (1,5 ml) et on a ajouté 20 µl de billes magnétiques commercialisées sous la dénomination Dynal auxquelles était couplée de la streptavidine. On a laissé le tube subir des retournements sur une roue à environ 4°C pendant 15 min.

On a repris les cellules et les billes sur lesquelles ces cellules étaient fixées dans environ 4 µl de milieu complet additionné d'ADCM (procuré par le Centre de transfusion de Lyon, France), et on a ensuite distribué les cellules ainsi traitées sur une plaque de titrage comportant 24 puits pour culture cellulaire (à raison d'environ 2.10⁵ cellules par puits).

On a laissé les cellules incuber pendant environ 1 heure 30 minutes à 37°C. On a ensuite repris la totalité du contenu d'un puits dans un tube d'Eppendorf de 1,5 ml et on a centrifugé pendant 10 min à 2000 tours/min.

On a repris le produit de la centrifugation pour enlever le surnageant et plonger le culot de centrifugation dans l'azote liquide.

Les mutations ont été testées dans l'ADN, par les techniques moléculaires classiques, connues et couramment pratiquées par l'homme du métier.

L'analyse des résultats obtenus et de ceux des investigations menées en parallèle sur des gènes différents du V_{H}, et/ou avec des cellules autres que la BL2, montre que l'on peut ainsi induire des mutations in vitro dans la cellule BL2 sur le gène V_{H} réarrangé par stimulation d'un groupe de récepteurs de surface.

En variante, on peut remplacer la séquence Ig, par exemple par une séquence procaryote, telle que le gène de résistance à la néomycine, et obtenir des mutations similaires avec la BL2.

Il a ainsi pu être établi, selon l'invention, que:
- des mutations peuvent être induites en environ 1 h 30 min après stimulation par le récepteur à l'antigène et un groupe de co-récepteurs, en l'absence de co-culture avec des cellules T;
- en moyenne un tiers environ des cellules subissent le mécanisme d'hypermutation;
- la mutation induite selon l'invention a lieu en présence d'actinomycine D, qui inhibe totalement la transcription;
- les mutations sont induites lorsque les cellules sont maintenues en G0/G1 par l'hydroxyurée;
- les mutations sont également induites en environ 30 minutes lorsque les cellules sont préparées en phase G0/G1 par élutriation.

Ces résultats vont à l'encontre des enseignements de la technique antérieure et ils montrent que le processus d'hypermutation peut avoir lieu en l'absence de transcription et de chromatide soeur.

En pratique, on peut ainsi étudier en gel à deux dimensions la perte ou l'apparition de protéines, mais aussi leur modification post-traductionnelle.

Par ailleurs, des mutations somatiques ainsi réalisées selon la présente invention ont pu être ré-induites dans des cellules appropriées, telles que le lymphome de Burkitt BL2, par stimulation des récepteurs de surface. Une stimulation de ce type se produit très vraisemblablement in vivo également.

Le procédé et les moyens selon l'invention permettent d'aborder dans des conditions radicalement plus performantes et sûres les études et tests de détermination des phénomènes d'hypermutation des gènes, en particulier des gènes des immunoglobulines.

Certains développements et certaines applications qui peuvent être visés grâce à la mise en oeuvre des moyens selon l'invention sont résumés ci-après. Il est clair que cette énumération n'est pas limitative et que d'autres applications également peuvent être envisagées.

A titre d'exemple, il est important que l'on puisse déterminer selon l'invention, dans des conditions qui ne pouvaient être réalisées jusqu'ici, quels sont le rôle et l'importance de l'hypermutation somatique dans la transformation maligne des cellules B dans le centre germinatif.

L'invention, en procurant un modèle unique de simplicité et de rapidité d'induction des mutations somatiques, a permis de montrer que le processus d'hypermutation peut avoir lieu en l'absence de transcription et de chromatide soeur.

Elle procure également des moyens pour l'obtention de la signature hypermutation au niveau protéique et génique, avec comme visée de permettre de disposer d'un test simple, applicable en particulier aux lymphomes B humains.

D'autres questions non résolues à ce jour sont notamment celle du mécanisme moléculaire de l'hypermutation et de l'éventuelle présence d'une polymérase hautement mutagène, ainsi que celle des cofacteurs présents.

Il faut en effet rappeler que les cofacteurs, c'est-à-dire les composants du mutasome qui permettent le ciblage du processus d'hypermutation sur les gènes V_{H} et V_{L} réarrangés à un stade précis de la réponse immune, ne sont pas connus. Le système de test selon l'invention procure des moyens fiables et rapides pour suivre la cinétique d'un tel processus d'hypermutation et pour que l'homme du métier, sur la base de ses connaissances propres et éclairé par la présente description, puisse réaliser des tests rapides et économiques notamment applicables aux lymphomes B humains.

Encore un autre objet de la présente invention est ainsi un kit ou ensemble de moyens pour l'induction d'hypermutations et pour la réalisation de tests y relatifs, dans lequel le moyen pour l'induction d'une hypermutation rapide comporte une combinaison d'anticorps telle que décrite plus haut.

De manière plus générale, la présente invention procure des moyens permettant l'étude et le suivi des premières étapes moléculaires des processus de mutation somatique au niveau de l'ADN et des protéines.

En bref, on peut ainsi, grâce aux moyens mis en oeuvre selon l'invention, modifier un gène par recombinaison homologue ou l'inactiver, par exemple dans la cellule BL2. La technique selon l'invention, ainsi rendue réalisable dans les cellules, notamment les cellules BL2, permet d'améliorer les propriétés mutatrices de ces cellules, mais également de tester le rôle que pourrait jouer telle ou telle molécule dans la physiologie des cellules B et des lymphomes de Burkitt.

En mettant en oeuvre les moyens selon l'invention on peut par conséquent, à titre d'exemples non limitatifs, obtenir la sur-expression de certains gènes dans la BL2 et améliorer ainsi les performances de cellule mutatrice de celle-ci, tandis que la transfection des molécules qui semblent impliquées dans le processus peut concerner, entre autres: AID, MSH2, MSH6 ou des enzymes de type RAD30A et/ou RAD30B.

Les mutations sont induites dans la phase G1 du cycle cellulaire et se produisent sur un brin d'ADN du gène V_{H} réarrangé, avec fixation possible par réplication dans l'une des cellules filles.

Les présents inventeurs ont par ailleurs également pu établir le rôle déterminant de pol iota, qui est une ADN polymérase de la famille Y, dans un tel processus d'hypermutation somatique (SHM) des gènes des immunoglobulines.

Pour produire des clones de BL2 déficients en pol iota (qui est l'un des deux homologues humains de la levure RAD30 présentant une activité de polymérase fortement sujette à des erreurs, de remplissage des courtes brèches d'ADN), on a utilisé un procédé d'inactivation de gènes semblable à celui qui a été développé pour l'inactivation du gène de l'AID (cytidine désaminase induite par activation, ou en anglais "Activation-Induced Cytidine Deaminase). La molécule AID a été décrite, notamment, par Muramatsu, M. et al., Class switch recombination and hypermutation require activation-induced cytidine deaminase (AID), a potential RNA editing enzyme, Cell 102, 553-63 (2000); et Revy, P. et al., Activation-induced cytidine deaminase (AID) deficiency causes thé autosomal recessive form of the Hyper-IgM syndrome (HIGM2), Cell 102, 565-75 (2000).

Deux clones pol iota^{-/-} (54 et 267) ont été générés à partir d'un clone hétérozygote de pol iota. Des constructions appropriées ont été utilisées pour inactiver les deux allèles. Les deux clones iota^{-/-} avaient la même vitesse de prolifération que la lignée cellulaire de BL2 originale, avec un indice mitotique et un profil de cycle cellulaire similaires, et ne présentaient pas d'aberrations chromosomiques ou de translocations. On n'a détecté aucune expression de pol iota dans les Western blots d'extraits cellulaires des clones iota^{-/-} 54 et 267, en utilisant des anticorps polyclonaux de lapin élevés contre pol iota humain. L'expression de pol iota a été restaurée par transfection de vecteurs pIRES commandant l'expression de l'ADNc de pol iota humain complet sous le contrôle du promoteur de CMV: dans les expériences effectuées, les taux d'expression étaient comparables à celui observé dans la lignée de cellules BL2 normales, dans les limites des variations inhérentes aux quantifications par Western blot. Après plusieurs tentatives, il n'a pas été possible d'obtenir de clones surexprimant pol iota, aussi bien sur un fond (background) normal que sur un fond déficient en pol iota. Deux clones restaurés pour chaque cellule cible ont été sélectionnés pour la suite de l'analyse.

On peut utiliser deux méthodes différentes pour induire dans la lignée cellulaire BL2 une hypermutation de son gène V_{H} réarrangé. La première fait intervenir une liaison croisée ou réticulation d'anti-IgM en co-culture avec un clone T auxiliaire, ou une lignée de cellules T auxiliaires, auquel cas lés mutations, si elles se produisent, sont observées après-3 jours en culture (voir Denépoux, S. et al., *supra;* et Poltoratsky, V. et al., Expression of error-prone polymerases in BL2 cells activated for Ig somatic hypermutation, Proc. Natl. Acad. Sci. USA 98, 7976-81 (2001)). Dans les présents essais, ce système d'induction de SHM a été adapté par utilisation de la lignée cellulaire CB15, qui est une cellule T auxiliaire transformée par l'herpèsvirus Saimiri, en co-culture, et une liaison croisée d'IgM suivie par l'agrégation de l'anticorps anti-IgM biotinylé, par des billes de streptavidine. La deuxième méthode ne fait intervenir que la réticulation ou liaison croisée des trois récepteurs de surface, IgM, CD19 et CD21, ou d'une combinaison d'IGM+CD19 et/ou d'IGM+CD21, suivie de la même agrégation de l'anticorps anti-IgM biotinylé, par des billes de streptavidine, auquel cas des mutations étaient observées après seulement 90 minutes d'incubation. La fréquence de mutation résultant d'un seul round de mutagenèse était semblable selon les deux méthodes, c'est-à-dire 4-6 x 10⁻⁴ par paire de bases (voir Tableau 1 plus loin). La lignée de cellules BL2 présente également une très faible fréquence de mutation constitutive de son gène V_{H} réarrangé, lorsqu'elle est maintenue en culture, en pratique, ne dépassant pas 10⁻⁴ mutations par paire de base après 2 à 3 mois en culture.

On n'a observé aucune augmentation détectable de la fréquence de mutation lorsqu'on a induit pour l'hypermutation les clones déficients en pol-iota 54 ou 267, aussi bien dans la culture de cellules T que dans l'essai avec trois anticorps susdit: on a seulement relevé 7 mutations parmi 298 séquences V après stimulation contre 5 parmi 222 séquences avant stimulation. Au contraire, l'induction des mutations a été restaurée par réexpression de l'ADNc de pol iota dans ces clones, à des niveaux comparables à ceux que présentait la BL2 normale: 83 mutations dans 547 séquences V après stimulation contre 6 dans 547 séquences V avant stimulation. En outre, le schéma de mutation restauré dans ces clones exprimant pol iota était similaire au schéma de mutation de la BL2, avec un ciblage élevé des paires de bases GC et une tendance pour les transitions (voir Tableau 2). Comme pour la BL2 normale, le gène Cµ des clones restaurés de pol-iota n'est pas apparu être ciblé pour la mutation après induction.

**Tableau 1**

| Fréquences de mutation de gène V dans des sous-clones de BL2 déficients en pol iota et compétent pour pol-iota | | | |
|---|---|---|---|
| Type de cellule | Non stimulée | Stimulée | |
| | | Anti-IgM/CD19/CD21 | Anti-IgM/CB15 |
| BL2 | 0,71 x 10⁻⁴ (7 / 236) | 3,7 x 10⁻⁴ (29 / 187) | 3,8 x 10⁻⁴ (26 / 165) |
| Clones pol iota^{-/-} | | | |
| 54 | 0,82 x 10⁻⁴ (3 / 88) | 0,96 x 10⁻⁴ (4 / 100) | 0,51 x 10⁻⁴ (2 / 95) |
| 267 | 0,36 x 10⁻⁴ (2 / 134) | 1,00 x 10⁻⁴ (1 / 24) | < 0,30 x 10⁻⁴ (0 / 79) |
| Clones pol iota^{-/-} restaurés avec ADNc de pol iota | | | |
| 54-7 | 0,31 x 10⁻⁴ (1 / 78) | 2,9 x 10⁻⁴ (14 / 115) | 3,1 x 10⁻⁴ (9 / 69) |
| 54-10 | 0,43 x 10⁻⁴ (2 /111) | 2,4 x 10⁻⁴ (8 / 79) | n.d. |
| 267-20 | 0,27 x 10⁻⁴ (1 / 89) | 4,0 x 10⁻⁴ (19 /113) | 5,1 x 10⁻⁴ (22 / 104) |
| 267-12 | 0,50 x 10⁻⁴ (2 / 97) | 4,0 x 10⁻⁴ (11 / 67) | n.d. |

| | | | |
|---|---|---|---|
| Nota: Les fréquences des mutations sont exprimées en mutation par nucléotide, avec le nombre total de mutations par gène V séquencé (416 pb) indiqué entre parenthèses. | | | |

**Tableau 2**

| Pourcentage de substitution de nucléotides dans des mutations générées dans le gène V4-39-JH5 de la lignée cellulaire pol iota^{-/-} de BL2 restaurée pour l'expression de pol iota^{a} | | | | | |
|---|---|---|---|---|---|
| A: | A | T | G | C | **Total** |
| De: | | | | | |
| A | | **3,6** (0,9) | **7,2** (4,6) | **1,8** (1,4) | **24,9** (20,5) |
| T | **4,6** (3,1) | | **0** (2,7) | **7,7** (7,8) | |
| G | **28,1** (26,3) | **13,5** (4,8) | | **1,3** (7,5) | **75,1** (79,5) |
| C | **9,6** (10,6) | **19,0** (24,2) | **3,6** (6,1) | | |
| Transitions: | **62,0** (62,9) | | Transversions: | **38,0** | (37,1) |
| | | | | | |

| | | | | | |
|---|---|---|---|---|---|
| Nota: Soixante-quatorze substitutions de bases ciblées dans 416 pb de la séquence VDJ ont été analysées, sur un total de 83 mutations comprenant 9 insertions (indiquées dans le Tableau 1). Le pourcentage de substitution est corrigé de la composition de bases du segment V. Les nombres en caractères gras concernent les mutations induites dans les clones pol iota restaurés, tandis que les valeurs relatives à l'induction de BL2 normal sont entre parenthèses. | | | | | |

Un autre objet de l'invention est l'utilisation de l'ADN polymérase iota pour l'ajustement de l'hypermutagénicité dans des tests in vitro d'hypermutagénicité au niveau protéique et/ou génique, en particulier sur des lymphomes B, notamment le lymphome de Burkitt BL2.

Encore un autre objet de l'invention est l'utilisation d'une ADN polymérase iota améliorée, notamment restaurée après changement d'au moins l'un de ses acides aminés naturels selon des techniques connues de l'homme du métier, pour l'augmentation de l'hypermutagénicité dans de tels tests. Selon l'invention, cette amélioration de pol iota est avantageusement effectuée par mutagenèse au hasard du domaine II annulaire (« Ring Finger ») pour y remplacer au moins un acide aminé, puis restauration des fonctionnalités de l'enzyme.

L'invention a en outre pour objet un kit pour l'induction de mutations somatiques tel que décrit plus haut, et comportant en outre des moyens pour l'inactivation du gène pol iota et/ou pour la restauration de celui-ci par expression d'ADNc de pol iota.

L'identification de cette contribution de pol iota est illustrée plus en détail dans la partie expérimentale ci-après.

### Induction d'hypermutation du gène V dans la lignée de cellules BL2:

Une hypermutation du gène V a été induite selon deux modes opératoires différents. En utilisant le premier mode opératoire, on a effectué des liaisons croisées des récepteurs de surface IgM, CD19 et CD21 conformément au protocole décrit plus haut. Le deuxième mode d'activation reposait sur une co-culture avec la lignée cellulaire CB15, qui est une lignée de cellules T auxiliaires transformées par l'herpèsvirus de Saimiri. La CB15 a été cultivée dans du milieu RPMI 1640 (Invitrogen), complété avec 10% de sérum de veau foetal (Hyclone), de la pénicilline/streptomycine (Invitrogen) et 50 unités/ml d'IL2 (Sigma). La CB15 a été irradiée à 4000 rads, remise en suspension dans du milieu complet plus 2% de Caryoser (CTS, Lyon) et déposée à raison de 500.000 cellules par puits dans des plaques à 24 puits ou alvéoles qui avaient été revêtues pendant une nuit avec de l'anti-CD3 (OKT3, Janssen-Cilag, 2,5 µg/puits). On a incubé 2 x 10⁶ cellules BL2 dans 0,5 ml de RPMI, pendant 20 minutes sur de la glace, avec un anticorps anti-IgM biotinylé (0,6 mg/ml, Caltag Laboratories), puis on a réticulé avec des billes magnétiques conjuguées à de la streptavidine (Dynabeads M280, Dynal), pendant 20 minutes, sur un plateau tournant et à basse température. La BL2 a été remise en suspension à raison de 100.000 cellules/ml dans du milieu RPMI complété avec 2% de Caryoser, et on a déposé 0,5 ml par puits d'une plaque à 24 puits contenant CB15 irradiée et l'anti-CD3 (dans un rapport BL2:CB15 de 1:10). Les cellules ont été prélevées après 3 jours d'incubation à 37°C, l'ADN a été extrait au moyen d'un kit approprié, dénommé DNeasy tissue kit (Qiagen), et les mutations ont été analysées après amplification du gène V4-39-JH5. Les mutations ont été identifiées par des alignements multiples d'électrophérogrammes et utilisation du logiciel AutoAssembler (Applera).

### Inactivation du gène pol iota dans la BL2:

La séquence génomique de pol iota humaine a été recueillie à partir du clone de HTGS de 180 kb AC021325, qui contient tous les exons codants. Les fragments d'ADN utilisés pour générer les constructions de ciblage ont été amplifiés à partir d'ADN génomique de BL2 et clonés d'abord avec le kit de clonage pour ACP TOPO-XL (Invitrogen), excisés au moyen de Sall et XhoI si leurs sites étaient ajoutés aux amorces d'ACP, ou sous la forme de fragments MluI-NotI si des sites de restriction n'étaient pas indus, et clonés dans les sites correspondants d'un vecteur pBSK (Stratagene) auquel les sites SfiI et MluI avaient été ajoutés dans le site SacI de son polylieur. Des gènes de résistance à la néomycine ou à l'hygromycine ont été insérés entre les fragments encadrant 5' et 3'. Les amorces d'ACP suivantes ont été utilisées pour l'amplification:
Premier allèle:
   fragment 5', 5'-iotal-5', CACTCGAGTACCAGCCGTCTGGTGTTTG, et 5'-iotal-3', CAGTCGACTACAGTCTCCAGTCGCTC (3,1 kb);
   fragment 3', 3'-iotal-5', CACTCGAGCTCAAATCCAGAGCTAAAAG, et 3'-iotal-3', CAGTCGACATAGTTGCAGGTAACCACC (2,5 kb).
Deuxième allèle:
   fragment 5', 5'-iota2-5', CACTCGAGAGAGCGACTGGAGACTGTAG, et 5'-iota2-3', ACGTCGACAGGAAGAATGCAGAGTGACG (1,8 kb);
   fragment 3', 3'-iota2-5', CTCAGTCGACGGTGGTTACCTGCAACTATG, et 3'-iota2-3', CCAAGTCTCTCAACAACTGG (3,8 kb).

On a utilisé les polymérases Pfu turbo (Stratagene) pour l'amplification du fragment 5' de la première construction et Herculase (Stratagene) pour le fragment 3' de la deuxième construction (30 s à 94°C, 30 s à 62°C et 12 min à 68°C pendant 40 cycles), le système d'ACP Expand Long Template PCR system (Roche) pour le fragment 3' de la première construction (40 cycles conformément aux conditions du fournisseur, comprenant un incrément dans le temps d'élongation), et le kit d'ACP Advantage 2 PCR kit (Clontech) pour le fragment 5' de la deuxième construction (30 s à 94°C, 30 s à 64°C et 4 min à 68°C). La transfection de 30 µg de construits linéarisés par NotI ou MluI a été effectuée comme décrit par Bertocci, B. et al., Immunity 9, 257-65 (1998). Le criblage des clones ciblés du gène a été effectué par ACP sur des pools de 10 clones avec les amorces suivantes situées à l'extérieur de la construction et à l'intérieur du gène de résistance à la néomycine ou à l'hygromycine:
premier allèle, iota-5'-test, ATGACCCAAGCTCAAGACAGC
   et néo^{R}: CATAGCGTTGGCTACCCGTG dans 5', et 3'-iota2-3' et néo^{R} réverse, CACGGGTAGCCAACGCTATG dans 3';
deuxième allèle, iota-5'-test
   et hygro^{R}, GCTGTGTAGAAGTACTCGCC
   (Expand Long Template PCR system, Roche).

Pour le premier allèle, la recombinaison homologue a été obtenue dans un clone sur 518 transfectants.

Pour le deuxième allèle, 2 clones sur 535 étaient correctement ciblés.

On a attribué cette fréquence faible à l'utilisation d'enzymes d'ACP à fidélité médiocre dans la construction des vecteurs de ciblage. Une telle activité de polymorphisme spécifique d'une souche sur la recombinaison homologue a été observée dans les cellules ES murines, où une différence de séquence de 0,6% s'est avérée résulter en une division par 50 de l'efficacité de ciblage. Aussi, les autres inactivations de gènes effectuées depuis sur la BL2 avec des constructions amplifiées seulement avec l'ADN polymérase Pfu turbo (Stratagene), qui est une enzyme dont la fréquence d'erreur est inférieure à 10⁻⁴ par paire de bases dans les conditions d'amplification utilisées, ont donné une fréquence de ciblage dans l'intervalle de 1%-2%. Transfection de clones de BL2 déficients en pol iota:

Des clones déficients en pol iota ont été transfectés avec des vecteurs d'expression de pol iota, construits par amplification d'un ADNc de pol iota humaine de longueur totale (McDonald, J.P. et al., Genomics 60, 20-30 (1999)) avec les amorces suivantes:
5'-iota, GCGGATCCACGACGAGGAAGACG, et
3'-iota, GCGGATCCTACGCTTTGTGCCAG
(site de BamHI souligné), dans le site BamHI du vecteur pIRES puro (Invitrogen). La transfection et la sélection des clones ont été effectuées conformément aux techniques classiques, connues de l'homme du métier, comme décrit plus haut.

### Analyse de clones de BL2 par RT-ACP et Western blot:

Des Western blots ont été effectués classiquement, avec des anticorps polyclonaux contre pol iota (peptide KLH-conjugué 15-mère, à extrémité C-terminale).

Il s'est en outre avéré que pol iota peut encore être améliorée selon des techniques connues de l'homme du métier (voir notamment technique de Prakash), mises au point pour l'enzyme pol éta, qui possède comme pol iota un domaine II annulaire (« Ring Finger ») dans lequel on peut faire muter, par une mutagenèse au hasard du dit domaine II, au moins un acide aminé présent dans ce domaine «Ring Finger », avec pour résultat une augmentation significative de l'activité de l'enzyme ainsi mutée.

L'invention a ainsi également pour objet un kit pour l'induction de mutations somatiques tel que décrit plus haut, et comportant une enzyme pol iota ainsi améliorée par mutagenèse au hasard de son domaine II annulaire pour y changer au moins un acide aminé avec pour résultat d'en améliorer les performances, puis restauration de l'enzyme par des moyens tels que ceux indiqués plus haut.

Ce système, dans ses diverses variantes de réalisation exposées plus haut, permet ainsi notamment d'identifier les mutations nécessaires pour améliorer les performances d'une protéine d'intérêt. Par exemple, dans le cas illustratif, et non limitatif, du gène codant pour l'insuline, mis dans une cassette promoteur-activateur transfectée dans la BL2, celle-ci peut produire de nombreuses molécules, parmi lesquelles celle ayant la meilleure performance va venir se fixer préférentiellement sur son substrat. En procédant ensuite au séquençage du gène muté codant pour cette protéine préférentielle et à l'identification par des moyens connus des acides aminés modifiés qui ont permis cette amélioration de performance, on peut identifier très rapidement par le procédé selon l'invention les mutations qui permettent d'obtenir une insuline plus performante.

## Revendications

1. Procédé pour l'induction de mutations somatiques in vitro, **caractérisé en ce qu'**il comporte (1) l'adjonction aux cellules à faire muter, dans des conditions de culture et dans un milieu appropriés aux dites cellules, d'au moins une combinaison d'anticorps comportant des anticorps anti-IgM choisie parmi une combinaison des anticorps anti-IgM et anti-CD19, une combinaison des anticorps anti-IgM et anti-CD21, et une combinaison des anticorps anti-IgM, anti-CD19 et anti-CD21, tandis que (2) lesdits anticorps anti-IgM sont biotinylés et sont soumis à une agrégation spécifique par la streptavidine couplée à un support.

2. Procédé selon la revendication 1, **caractérisé en ce que** ledit support est composé en totalité ou en partie par des billes magnétiques.

3. Procédé selon l'une quelconque des revendications 1 ou 2, **caractérisé en ce qu'**on utilise le lymphome de Burkitt BL2 pour les cellules à faire muter.

4. Procédé selon l'une quelconque des revendications 1 à 3, **caractérisé en ce qu'**il comporte en outre l'étape consistant à faire varier le taux de l'ADN polymérase iota.

5. Procédé selon l'une quelconque des revendications 1 à 4, **caractérisé en ce qu'**on restaure l'ADN polymérase iota préalablement inactivée, par transfection de vecteurs pIRES conduisant l'expression de l'ADNc de pol iota complète sous le contrôle du promoteur de CMV.

6. Utilisation du procédé selon l'une quelconque des revendications 1 à 5, pour la réalisation de tests in vitro d'hypermutagénicité au niveau protéique et/ou génique.

7. Utilisation selon la revendication 6, **caractérisée en ce qu'**elle est appliquée aux lymphomes B, en particulier aux lymphomes B humains.

8. Utilisation selon la revendication 7, **caractérisée en ce qu'**elle est destinée à l'induction de mutations sur le lymphome de Burkitt BL2.

9. Utilisation selon la revendication 8, **caractérisée en ce qu'**elle vise l'induction de mutations sur le lymphome de Burkitt BL2 dans la phase G0/G1.

10. Utilisation selon l'une quelconque des revendications 6 à 9, **caractérisée en ce qu'**elle comporte en outre l'utilisation de l'ADN polymérase iota pour l'ajustement de l'hypermutagénicité dans des tests in vitro d'hypermutagénicité au niveau protéique et/ou génique, en particulier sur des lymphomes B, notamment le lymphome de Burkitt BL2.

11. Utilisation selon la revendication 10, **caractérisée en ce qu'**on met en oeuvre une ADN polymérase iota avec remplacement d'au moins un acide aminé dans le domaine II Ring finger.

12. Utilisation d'un kit comportant des moyens pour l'induction de mutations somatiques comprenant au moins une combinaison d'anticorps comportant des anticorps anti-IgM choisie parmi une combinaison des anticorps anti-IgM et anti-CD19, une combinaison des anticorps anti-IgM et anti-CD21, et une combinaison des anticorps anti-IgM, , anti-CD19 et anti-CD21, pour l'induction de mutations somatiques, in vitro.

13. Kit pour utilisation selon la revendication 12, **caractérisé en ce que** lesdits anticorps anti-IgM sont biotinylés et ont été soumis à une agrégation spécifique, par la streptavidine couplée à un support, notamment à des billes magnétiques, tandis que le kit comprend une combinaison ternaire des anticorps anti-IgM, anti-CD19 et anti-CD21.

14. Kit pour utilisation selon l'une des revendications 12 ou 13, **caractérisé en ce qu'**il comporte en outre des moyens pour l'inactivation du gène pol iota et/ou pour la restauration de celui-ci par expression d'ADNc de pol iota.

15. Kit selon la revendication 14, **caractérisé en ce qu'**il comporte une ADN polymérase iota avec remplacement d'au moins un acide aminé, dans le domaine II Ring finger.

16. Utilisation selon la revendication 12 pour l'identification qualitative et/ou quantitative de composants du mutasome, notamment par l'analyse des protéines.

17. Utilisation selon la revendication 16, **caractérisée en ce qu'**elle comporte l'identification, des modifications post-traductionnelles induites, en particulier par identification, isolement et analyse d'un composant du, mutasome, notamment d'un composant protéinique, qui apparaît pendant ladite induction.

18. Utilisation selon la revendication 16, **caractérisée en ce qu'**elle comporte la fourniture d'au moins un gène, notamment un gène codant pour une protéine d'intérêt, sur lequel on souhaite induire des mutations, tandis que ledit gène est inclus dans une cassette contenant le promoteur et l'activateur (enhancer) des gènes codant pour la chaîne lourde ou légère des IgG, et que ledit gène est transfecté dans des cellules de lymphome, notamment de lymphome de Burkitt BL2.

19. Utilisation selon l'une quelconque des revendications 16 à 18, **caractérisée en ce que**, pour faire muter une séquence quelconque, on encadre la séquence à faire muter par le promoteur et l'activateur des Ig, de préférence dans une cassette de mutation.

20. Utilisation selon l'une quelconque des revendications 16 à 18, **caractérisée en ce que**, pour faire muter une séquence quelconque, on insère cette séquence par recombinaison homologue au locus des immunoglobulines, en remplacement de tout ou partie du gène V d'un des deux loci réarrangés.

## Claims

1. Process for the induction of somatic mutations in vitro, **characterized in that** it comprises (1) the addition to the cells to be mutated, under culturing conditions and in a medium that are suitable for said cells, of a least one combination of antibodies comprising anti-IgM antibodies selected from a combination of anti-IgM and anti-CD19 antibodies, a combination of anti-IgM and anti-CD21 antibodies and a combination of anti-IgM, anti-CD19 and anti-CD21 antibodies, while (2) said anti-IgM antibodies are biotinylated and are subjected to specific aggregation by streptavidin coupled to a support.

2. Process according to claim 1, **characterized in that** said support is composed, entirely or in part, of magnetic beads.

3. Process according to claims 1 or 2, **characterized in that** the BL2 Burkitt's lymphoma is used for the cells to be mutated.

4. Process according to any one of claims 1 to 3, **characterized in that** it further comprises the step consisting of adjusting the level of iota DNA polymerase.

5. Process according to any one of the claims 1 to 4, **characterized in that** the previously inactivated iota DNA polymerase is restored by transfection of pIRES vectors, leading to the expression of complete Pol iota cDNA under the control of the CMV promoter.

6. Use of the process according to any one of claims 1 to 5 for carrying out *in vitro* hypermutagenicity tests at the protein and/or genic level.

7. Use according to claim 6, **characterized in that** it is applied to B lymphomas, in particular to human B lymphomas.

8. Use according to claim 7, **characterized in that** it is designed for the induction of mutations in BL2 Burkitt's lymphoma.

9. Use according to claim 8, **characterized in that** it aims at the induction of mutations in BL2 Burkitt's lymphoma in the G0/G1 phase.

10. Use according to any claims 6 to 9, **characterized in that** it additionally comprises the use of iota DNA polymerase for adjusting the hypermutagenicity in *in vitro* hypermutagenicity tests carried out at the protein and/or genic level, in particular in B lymphomas, notably in BL2 Burkitt's lymphoma.

11. Use according to claim 10, **characterized in** using iota DNA polymerase with replacement of at least one amino acid in the ring finger domain II.

12. Use of a kit, **characterized in that** it comprises means for the induction of somatic mutations, comprising at least one combination of antibodies comprising anti-IgM antibodies selected from a combination of anti-IgM anti-CD19 antibodies, a combination of anti-IgM and anti-CD21 antibodies, and a combination of anti-IgM, anti-CD19 and anti-CD21 antibodies, for the induction of somatic mutations in vitro.

13. Kit for use according to claim 12, **characterized in that** said anti-IgM antibodies are biotinylated and have been subjected to specific aggregation by streptavidin coupled to a support, notably to magnetic beads, whereas the kit comprises a ternary combination of anti-IgM, anti-CD19 and anti-CD21 antibodies.

14. Kit for use according to either of claims 12 or 13, **characterized in that** it additionally comprises means for inactivation of the Pol iota gene and/or for the restoration thereof by Pol iota cDNA expression.

15. Kit according to claim 14, **characterized in that** it comprises iota DNA polymerase with replacement of at least one amino acid in the ring finger domain II.

16. Use according to claim 12 for qualitative and/or quantitative identification of components of the mutasome, in particular by protein analysis.

17. Use according to claim 16, **characterized in that** it comprises identification of the induced post-translational modifications, carried out, in particular, by identification, isolation and analysis of a component of the mutasome, notably of a protein component, which appears during said induction.

18. Use according to claim 16, **characterized in that** it comprises the provision of at least one gene, notably a gene coding for a protein of interest, in which it is desired to induce mutations, while said gene is included in a cassette containing the promoter and activator (enhancer) of the genes coding for the heavy or light chain of the IgG's, and that said gene is transfected in lymphoma cells, in particular in BL2 Burkitt's lymphoma cells.

19. Use according to any one of claims 16 to 18, **characterized in that**, in order to cause mutation of any given sequence, said sequence to be mutated is surrounded by the promoter and activator of the Ig, preferably in a mutation cassette.

20. Use according to any of claims 16 to 18, **characterized in that**, in order to mutate any given sequence, this sequence is inserted by homologous recombination at the locus of the immunoglobulins, replacing all or part of the V gene of one of the two rearranged loci.

## Patentansprüche

1. Verfahren für die Induktion somatischer Mutationen in vitro, **dadurch gekennzeichnet, dass** es (1) die Zugabe zu den zu mutierenden Zellen unter Kulturbedingungen und in einem für diese Zellen geeigneten Medium mindestens einer Kombination von Antikörpern, umfassend Anti-IgM-Antikörper, umfasst, die aus einer Kombination von Anti-IgM- und Anti-CD19-Antikörpern, einer Kombination von Anti-IgM-und Anti-CD21-Antikörpern und einer Kombination von Anti-IgM-, Anti-CD19- und Anti-CD21-Antikörpern ausgewählt ist, wobei (2) die Anti-IgM-Antikörper biotinyliert sind und einer spezifischen Aggregation mit Streptavidin, das an einen Träger gebunden ist, unterzogen wurden.

2. Verfahren nach Anspruch 1, **dadurch gekennzeichnet, dass** der Träger in seiner Gesamtheit oder teilweise aus Magnetkugeln besteht.

3. Verfahren nach einem der Ansprüche 1 oder 2, **dadurch gekennzeichnet, dass** man Burkitt-Lymphom BL2 als die zu mutierenden Zellen verwendet.

4. Verfahren nach einem der Ansprüche 1 bis 3, **dadurch gekennzeichnet, dass** es ferner den Schritt eines Variierens des Anteils der DNA-Polymerase iota umfasst.

5. Verfahren nach einem der Ansprüche 1 bis 4, **dadurch gekennzeichnet, dass** man die DNA-Polymerase iota, die zuvor inaktiviert wurde, durch Transfektion von pIRES-Vektoren, die cDNA des vollständigen Pol iota unter der Kontrolle des CMV-Promotors exprimieren, wiederherstellt.

6. Verwendung des Verfahrens nach einem der Ansprüche 1 bis 5 für die Durchführung von in-vitro-Tests einer Hypermutagenizität auf Protein- und / oder Genebene.

7. Verwendung nach Anspruch 6, **dadurch gekennzeichnet, dass** sie auf B-Lymphome, insbesondere auf menschliche B-Lymphome angewendet wird.

8. Verwendung nach Anspruch 7, **dadurch gekennzeichnet, dass** sie zur Induktion von Mutationen des Burkitt-Lymphoms BL2 bestimmt ist.

9. Verwendung nach Anspruch 8, **dadurch gekennzeichnet, dass** sie auf die Induktion von Mutationen des Burkitt-Lymphoms BL2 in der G0/G1-Phase gerichtet ist.

10. Verwendung nach einem der Ansprüche 6 bis 9, **dadurch gekennzeichnet, dass** sie ferner die Verwendung der DNA-Polymerase iota für die Anpassung der Hypermutagenizität in in-vitro-Tests einer Hypermutagenizität auf Protein- und / oder Genebene, insbesondere bei B-Lymphomen, insbesondere bei dem Burkitt-Lymphom BL2, umfasst.

11. Verwendung nach Anspruch 10, **dadurch gekennzeichnet, dass** man eine DNA-Polymerase iota mit einem Austausch mindestens einer Aminosäure in der Ring-Finger-Domäne II verwendet.

12. Verwendung eines Kits, umfassend Mittel für die Induktion von somatischen Mutationen, die mindestens eine Kombination von Antikörpern, umfassend Anti-IgM-Antikörper, umfassen, die aus einer Kombination von Anti-IgM- und Anti-CD19-Antikörpern, einer Kombination von Anti-IgM- und Anti-CD21-Antikörpern und einer Kombination von Anti-IgM-, Anti-CD19- und Anti-CD21-Antikörpern ausgewählt ist, für die Induktion somatischer Mutationen in vitro.

13. Kit für eine Verwendung nach Anspruch 12, **dadurch gekennzeichnet, dass** die Anti-IgM-Antikörper biotinyliert sind und einer spezifischen Aggregation mit Streptavidin, das an einen Träger, insbesondere an Magnetkugeln, gebunden ist, unterzogen wurden, wobei der Kit eine Dreierkombination von Anti-IgM-, Anti-CD19- und Anti-CD21-Antikörpern umfasst.

14. Kit für eine Verwendung nach einem der Ansprüche 12 oder 13, **dadurch gekennzeichnet, dass** er ferner Mittel für die Inaktivierung des Gens von Pol iota und / oder für die Wiederherstellung davon durch eine Expression der cDNA von Pol iota umfasst.

15. Kit nach Anspruch 14, **dadurch gekennzeichnet, dass** er eine DNA-Polymerase iota mit einem Austausch mindestens einer Aminosäure in der Ring-Finger-Domäne II umfasst.

16. Verwendung nach Anspruch 12 für die qualitative und/oder quantitative Identifizierung der Bestandteile des Mutasoms, insbesondere durch die Analyse von Proteinen.

17. Verwendung nach Anspruch 16, **dadurch gekennzeichnet, dass** sie die Identifizierung von induzierten post-translationalen Modifikationen insbesondere durch Identifizierung, Isolierung und Analyse eines Bestandteiles des Mutasoms, insbesondere eines Proteinbestandteiles, der während der Induktion auftritt, umfasst.

18. Verwendung nach Anspruch 16, **dadurch gekennzeichnet, dass** sie die Bereitstellung mindestens eines Gens, insbesondere eines Gens, das für ein Protein von Interesse kodiert, bei dem Mutationen induziert werden sollen, umfasst, wobei das Gen in einer Kassette eingeschlossen ist, die den Promotor und den Aktivator (Enhancer) von Genen enthält, die für die schwere oder leichte Kette des IgG kodieren, und das Gen in Lymphomzellen, insbesondere Zellen des Burkitt-Lymphoms BL2, transfiziert wurde.

19. Verwendung nach einem der Ansprüche 16 bis 18, **dadurch gekennzeichnet, dass** man zum Mutieren einer beliebigen Sequenz die zu mutierende Sequenz mit dem Ig-Promotor und -Aktivator, vorzugsweise in einer Mutationskassette, versieht.

20. Verwendung nach einem der Ansprüche 16 bis 18, **dadurch gekennzeichnet, dass** man zum Mutieren einer beliebigen Sequenz die Sequenz durch homologe Rekombination in den Locus der Immunoglobuline einfügt, indem man das ganze oder einen Teil des V-Gens eines der beiden umgelagerten Loci austauscht.
